# EUROPEAN PATENT SPECIFICATION

(11) **EP 2 633 632 B1**
(45) Date of publication and mention of the grant of the patent: **23.06.2021**
(21) Application number: 11836593.1
(22) Date of filing: 25.10.2011
(51) Int. Cl.: H04B 5/00, H04W 4/80, G16H 10/60, A61B 5/00

(54) **METHOD AND SYSTEM OF COMMUNICATING PERSONAL HEALTH DATA IN A NEAR FIELD COMMUNICATION ENVIRONMENT**
VERFAHREN UND SYSTEM ZUR ÜBERMITTLUNG VON PATIENTENGESUNDHEITSDATEN IN EINER NAHFELDKOMMUNIKATIONSUMGEBUNG
PROCÉDÉ ET SYSTÈME DE COMMUNICATION DE DONNÉES DE SANTÉ PERSONNELLES DANS UN ENVIRONNEMENT DE COMMUNICATION EN CHAMP PROCHE

(30) Priority: 25.10.2010 IN 3173CH2010
(43) Date of publication of application: 04.09.2013
(60) Divisional application: 14186526.1
(73) Proprietor: Samsung Electronics Co., Ltd., Gyeonggi-do 443-742 (KR)
(72) Inventor: REDDY BADVEL, Jayabharath, BANGALORE 560093 (IN); ARUNAN, Thenmozhi, BANGALORE 560093 (IN); WON, Eun-Tae, Gyeonggi-do 443-742 (KR)
(74) Representative: Nederlandsch Octrooibureau
(86) International application number: PCT/KR2011/007981
(87) International publication number: WO 2012/057501

(56) References cited:
- EP-A2- 2 573 970
- WO-A1-2009/068931
- US-A1- 2008 162 312
- US-A1- 2008 220 878
- US-A1- 2010 004 950
- US-A1- 2010 041 332
- US-A1- 2010 041 332
- US-A1- 2010 045 425
- US-A1- 2010 169 686
- "Android NDEF Push Protocol Specification", , 22 February 2011 (2011-02-22), XP055163343, Retrieved from the Internet: URL:http://static.googleusercontent.com/ex ternal_content/untrusted_dlcp/source.andro id.com/en//compatibility/ndef-push-protoco l.pdf [retrieved on 2015-01-20]
- "Simple NDEF Exchange Protocol - Technical Specification, SNEP 1.0", , 31 August 2011 (2011-08-31), pages 1-20, XP055081363, Wakefield, MA, USA 01880 Retrieved from the Internet: URL:http://www.jortec.neec-fct.org/wp-cont ent/uploads/2013/02/Simple_NDEF_Exchange_P rotocol.pdf [retrieved on 2013-09-27]
- "Health Informatics-Personal Health Device Communication Part 20601: Application ProfileOptimized Exchange Protocol;IEEE Std 11073-20601-2008", IEEE STANDARD, IEEE, PISCATAWAY, NJ, USA, 19 December 2008 (2008-12-19), pages c1-198, XP017604162, ISBN: 978-0-7381-5826-6

## Description

### Technical Field

The present invention relates to the field of near field communication system, and more particularly relates to a method and system of communicating personal health data in a near field communication environment.

### Background Art

Near Field Communication (NFC) is used in devices for communicating with other devices in a network range of less than 10cm. Typically, in NFC ecosystem, user applications can read or write information from or to NFC tags. NFC tags are static in nature and do not have capabilities for dynamically processing of data stored in it. Generally, NFC tags are powered by radio frequency field generated by an active NFC device and are able to respond to requests from the active NFC device.

ISO/IEEE 11073 standards enable communication between medical devices and external systems. Personal health devices which are complaint with ISO/IEEE 11073 standards can communicate with each other using ISO/IEEE 11073-20601 communication protocol. ISO/IEEE 11073 standard defines 'agent' as a node that collects and transmits personal health data to an associated NFC manager and 'manager' as a node receiving personal health data from one or more agents. Exemplary manager includes cell phones, health appliance, set top box, personal computer system and the like. ISO/ IEEE 11073-20601 standard defines communication protocol between IEEE 11073 Agent and IEEE 11073 Manager.

Typically, a NFC manager (i.e., IEEE 11073 Manager with a NFC Read/Write Interface) and a NFC agent (i.e., IEEE 11073 Agent with a NFC Tag) communicate in a NFC Reader/Writer mode using a NFC Data Exchange Format (NDEF) message. NDEF message can be of type, text, URI, image, MIME type, etc. In NFC reader/ writer mode of communication, NDEF messages are exchanged between the NFC Manager and NFC Agent using the tag transport protocols.

Generally, the ISO/IEEE 11073 Agent and Manager exchange a sequence of ISO/ IEEE 11073-20601 APDUs for association, configuration and exchanging measurement data. This involves series of request and response exchanges between the Agent and the Manager US2010169686 A1 describes a communication interface between an NFCH and an NFC device.

US2010045425 A1 describes a method for data transmission between a sensor module for measuring and storing data and a mobile device using NFC.

### Disclosure of Invention

### Technical Problem

Usually, when a NFC manager writes a request into a NFC tag residing in an NFC agent, the NFC agent reads the request stored in the NFC tag and writes a response to the request into the NFC tag. The NFC manager reads the response written by the NFC agent from the NFC tag. However, in case the NFC agent delays reading the request from the NFC tag, the NFC manager reads the request written by itself and considers the request as a response from the NFC agent due to limited capabilities of the NFC tag, leading to connection set up latency and communication overhead.

### Solution to Problem

Aspects of the present invention are to address the above-mentioned problems and/or disadvantages and to provide at least the advantages described below. Accordingly, an aspect of the present invention is to provides a method and system for communicating personal health data in a near field communication (NFC) environment.

In accordance with an aspect of the present invention, a method of communicating personal health data in a near field communication (NFC) environment is provided. The method includes setting control information in a NFC data exchange format (NDEF) record by a first NFC device to synchronize communication between the first NFC device and a second NFC device in a NFC environment, wherein the control information includes at least one of a direction flag, a request/response type flag, a status flag and a sequence identifier, and writing the NDEF record containing the control information into a NFC tag.

In accordance with another aspect of the present invention, an apparatus is provided. The apparatus includes a processor, and memory coupled to the processor, wherein the memory comprises a read/write module configured for setting control information in a NFC data exchange format (NDEF) record, wherein the control information includes a direction flag, a request/response type flag, a status flag and a sequence identifier, and writing the NDEF record containing the control information into a NFC tag.

### Advantageous Effects of Invention

The present invention is to provides a method and system for communicating personal health data in a near field communication (NFC) environment.

### Brief Description of Drawings

Figure 1 is a block diagram of a near field communication (NFC) system enabling communication of personal health data between a NFC manager and an NFC agent, according to one embodiment.
Figure 2 is a process flowchart illustrating an exemplary method of reading/writing NFC data exchange format (NDEF) record into the NFC tag, according to one embodiment.
Figure 3a is a schematic representation of a NDEF record, according to one embodiment.
Figure 3b is a schematic representation of a payload field in the NDEF record, according to one embodiment.
Figure 4 is a schematic representation of a payload field in the NDEF record, according to another embodiment.
Figure 5 illustrates a block diagram of the NFC manager showing various components for implementing embodiments of the present subject matter.
Figure 6 illustrates a block diagram of the NFC agent showing various components for implementing embodiments of the present subject matter.

The drawings described herein are for illustration purposes only and are not intended to limit the scope of the present disclosure in any way.

### Best Mode for Carrying out the Invention

The present invention provides a method and apparatus for communicating personal health data in a near field communication (NFC) environment. In the following detailed description of the embodiments of the invention, reference is made to the accompanying drawings that form a part hereof, and in which are shown by way of illustration specific embodiments in which the invention may be practiced. These embodiments are described in sufficient detail to enable those skilled in the art to practice the invention, and it is to be understood that other embodiments may be utilized and that changes may be made without departing from the scope of the present invention. The following detailed description is, therefore, not to be taken in a limiting sense, and the scope of the present invention is defined only by the appended claims.

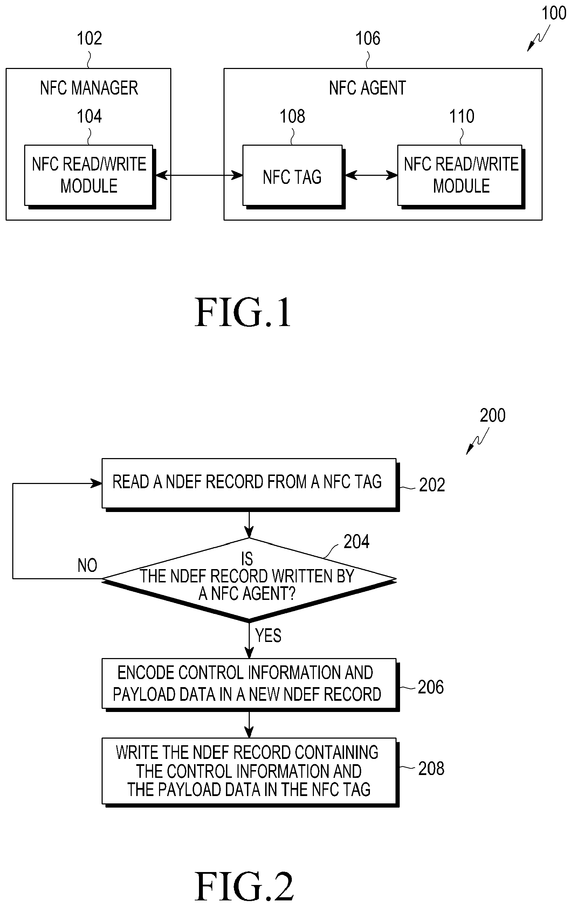

Figure 1 is a block diagram 100 of a near field communication (NFC) system enabling communication of personal health data between a NFC manager 102 and an NFC agent 106, according to one embodiment. Particularly, the NFC system 100 includes the NFC manager 102 having a NFC read/write module 104, and the NFC agent 106 with a NFC tag 108 and a NFC read/write module 110.

The NFC manager 102 may be a device, such as a cell phone, tablet, smart phone, personal digital assistant, set-top box, personal computer and the like, capable of communicating with the NFC agent 106. The NFC agent 106 is a device capable of collecting personal health data and communicating the personal health data with the NFC manager 102 via the NFC tag 108. The NFC manager 102 and the NFC agent 106 writes or reads data in/from the NFC tag 108 using ISO/IEEE 11073-20601 communication protocol.

For communicating personal health data, the NFC manager 102 and the NFC agent 106 exchanges a series of request/response messages via the NFC tag 108. In an exemplary operation, the NFC manager 102 sets control information in a NDEF (NFC data exchange format) record. The NDEF record may contain a header and a payload field. The payload field includes the control information and/or other payload data. It can be noted that, the control information can be encoded in other fields of the NDEF record other than the payload field. The control information may include a direction flag, a state flag, a request/response flag, and a sequence identifier. The direction flag indicates direction of communication of the NDEF record. The state flag indicates state of communication of the NFC manager 102 or the NFC agent 106. The request/ response flag indicates whether the NDEF record carries an ISO/IEEE 11073-20601 protocol request command or an ISO/IEEE 11073-20601 protocol response command. The sequence identifier indicates an identifier of NDEF records exchanged between the NFC manager 102 and the NFC agent 106. According to the present invention, the control information is set in the NDEF record to provide synchronized communication between the NFC manager 102 and the NFC agent 106 in order to efficiently obtain personal health data from the NFC agent 106. Upon setting the control information, the NFC read/write module 104 writes the NDEF record containing the control information and/or other payload data into the NFC tag 108.

Subsequently, the NFC read/write module 110 of the NFC agent 106 reads the NDEF record stored in the NFC tag 108. The NFC read/write module 110 retrieves the control information from the NDEF record and determines direction of communication, request/response command, a communication state, and a sequence identifier based on the direction flag, the request/response flag, the state flag, and the sequence identifier. In the above case, the direction flag indicates direction of communication is from the NFC manger 102 to the NFC agent 106. The request/response flag indicates that the NDEF record carries the request command in the payload field. The state flag indicates the communication state of the NFC manager when the NDEF record is written in the NFC tag 108. The sequence identifier indicates the sequence identifier allocated to the NDEF record by the NFC agent. Accordingly, the NFC read/write module 110 updates the control information (i.e., the direction flag, the sequence identifier, the state flag and the request/response flag) in the NDEF record and encodes payload data in the payload field of the NDEF record. Then, the NFC read/write module 110 writes the NDEF record in the NFC tag 108.

Substantially simultaneously, the NFC read/write module 104 reads the NDEF record containing the control information and other payload data from the NFC tag 108. The NFC read/write module 104 then retrieves the control information from the NDEF record and determines direction of communication, request/response command, a communication state, and a sequence identifier based on the direction flag, the request/ response flag, the state flag, and the sequence identifier. Accordingly, the NFC read/ write module 104 determines whether the NDEF record read from the NFC tag 108 is same as the previous NDEF record written into the NFC tag 108 based on the control information in the read NDEF record. For example, if the direction flag indicates the direction of communication as from the NFC manager 102 to the NFC agent 106 and the request/response flag indicates that the NDEF records contain the request command, then the NFC read/write module 104 identifies the NDEF record as same as the NDEF record previously written by the NFC manager 102 and not as the NDEF record received from the NFC agent 106. In such scenario, the read/write module 104 may ignore the read NDEF record and continue reading the NFC tag 108 after some time interval. It may happen that, the NDEF record read by the NFC read/write module 104 is same as the previously written NDEF record as the NDEF record written in the NFC tag 108 is not yet read by the NFC agent 106 or the NFC read/write module 104 has read the NFC tag 108 prior to writing a NDEF record by the NFC agent 106. In such a case, the control information in the NDEF record enables the NFC manager 102 determine the identity of the NDEF record, according to the present invention.

If the NFC read/write module 104 determines that the NDEF record contains payload data received from the NFC agent 106, then the NFC read/write module 104 processes the payload data and encodes new control information and payload data in a NDEF record. Then, the NFC read/write module 104 writes the NDEF record with the new control information and different payload data in the NFC tag 108. The above process continues till personal health data is communicated to the NFC manager 102 by the NFC agent 106 in the payload field of one of the NDEF records exchanged between the NFC manager 102 and the NFC agent 106.

Figure 2 is a process flowchart 200 illustrating an exemplary method of reading/ writing NDEF record into the NFC tag 108, according to one embodiment. At step 202, a NDEF record containing the control information and payload data is read from the NFC tag 108. As described above, the control information includes a direction flag, a state flag, a request/response flag, and a sequence identifier. At step 204, it is determined whether the NDEF record in the NFC tag 108 is written by the NFC agent 106 based on the control information. If the NDEF record is written by the NFC agent 106, then at step 206, the payload data in the read NDEF record is processed and new control information and payload data (e.g., IEEE 11073-20601 data) is encoded in payload field of a new NDEF record. At step 208, the new NDEF record containing the control information and the payload is written in the NFC tag 108. The steps 202-208 are repeated till complete ISO/IEEE 11073 personal health data is received from the NFC agent 106. If the NDEF record is written by the NFC manager 102, then the NDEF record is ignored and the NFC tag 108 is read again after some time to obtain another NDEF record. One skilled in the art will realize that the above steps 202-208 can also be implemented at the NFC agent 106.

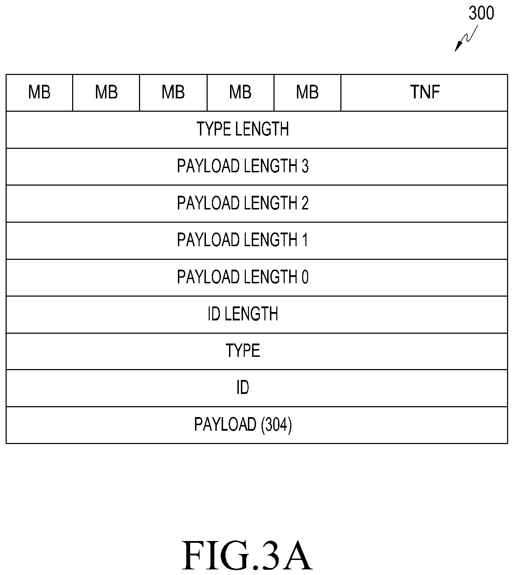

Figure 3a is a schematic representation of a NDEF record 300, according to one embodiment. The NFC record 300 includes a payload field 302. The payload field 302 includes control information associated with the NDEF record and other payload data, according to one embodiment. Alternatively, an ID field 304 includes control information associated with the NDEF record. The NDEF record 300 can be exchanged between the NFC manager 102 and the NFC agent 106 in peer to peer mode using a simple NDEF exchange protocol (SNEP). For example, the NFC manager 102 acting as a SNEP client can exchange the NDEF record 300 with the NFC agent 106 acting as SNEP server. For this, the NFC manager 102 can use SNEP request message with PUT request to send the NDEF record 300 to the NFC agent 106. The NFC agent 106 can send a response NDEF record to the NFC manager 102 using a SNEP response message with PUT request. Alternatively, the NFC manager 102 can use a SNEP request message with a GET request.

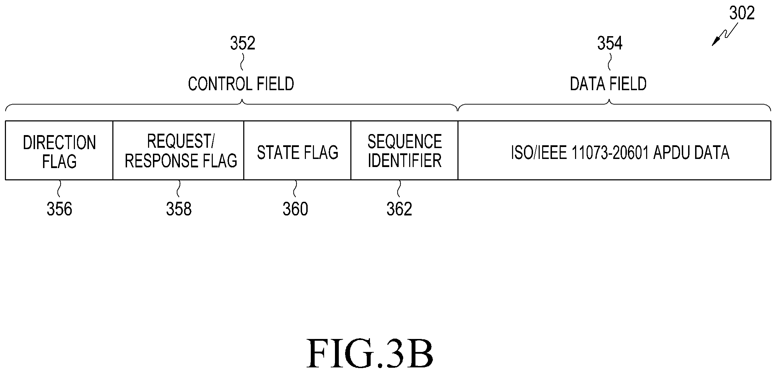

Figure 3b is a schematic representation 350 of the payload field 302 in the NDEF record 300, according to one embodiment. The payload field 302 includes a control field 352 and a data field 354. The control field 352 includes a direction flag field 356, a request/response flag field 358, a state flag field 360, and a sequence identifier field 362. The direction flag field 356 indicates direction of communication of the NDEF record. For example, the direction flag field 356 includes a value '0', if the NDEF record is written into the NFC tag 108 by the NFC agent 106. When the NDEF record is written by the NFC manager 102, the direction flag field 356 includes a value '1'.

The request/response flag field 358 indicates whether the NDEF format includes an ISO/IEEE 11073-20601 communication protocol request command or ISO/IEEE 11073-20601 communication protocol response command. For example, the request/ response flag field 358 includes a value '0' when the NDEF record includes a request command and a value '1' when the NDEF record includes a response command. The state flag field 360 indicates a communication state of a sender of the NDEF record. The state flag field 360 helps understand the state of the NFC manager 102 or the NFC agent 106 during a particular instance of communication. Exemplary values carried by the state flag field 360 to indicate a specific state of the NFC manager 102 or the NFC agent 106 is shown in table 1 below:

### Table 1

**[Table 1]**

| | |
|---|---|
| 0x01 | DISCONNECTED |
| 0x01 | CONNECTED |
| 0x02 | UNASSOCIATED |
| 0x03 | ASSOCIATING |
| 0x04 | ASSOCIATED |
| 0x05 | OPERATING |
| 0x06 | CONFIGURING |
| 0x07 | DISASSOCIATING |
| 0x08-0X3F | RFU |

For example, as shown in Table 1, the state flag field 360 may include a value '0x00' when the NFC agent 106 or the NFC manager 102 is in disconnected state. When the NFC agent 106 or the NFC manager 102 is in connected stated, the state flag field 362 will carry a value `0x01'.

The sequence identifier field 362 indicates a sequence identifier assigned to each NDEF record communicated between the NFC read/write module 104/110 and the NFC tag 108. In other words, the sequence identifier field 362 indicates the order in which the NDEF records are written in the NFC tag so that the NDEF records are not duplicated or missed during communication between the NFC manager 102 and the NFC agent 106. The sequence identifier, exchanged between the NFC manager 102 and the NFC agent 106, can be any random number or sequence of numbers incremented by one.

The sequence identifier field 362 enables controlling flow of NDEF records exchanged between the NFC read/write module 104/110 and the NFC tag 108. The sequence identifier in the sequence identifier field 362 provides reliability in communication between the NFC manager 102 and the NFC agent 106. It can be noted that, the sequence field 362 can also be included in a header of the NDEF record. The data field 354 includes ISO/IEEE 11073-20601 data exchanged between the NFC manager 102 and the NFC agent 106.

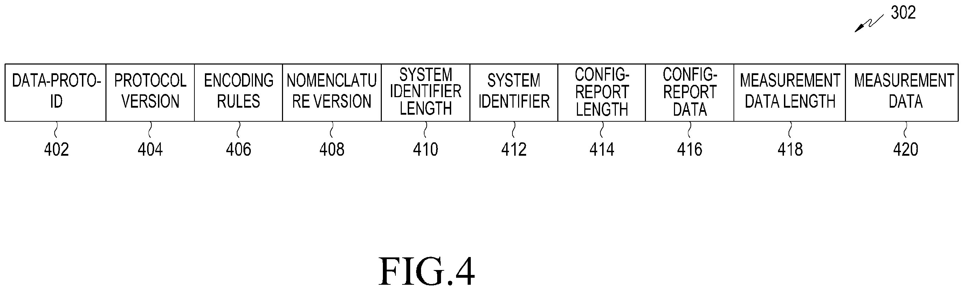

Figure 4 is a schematic representation of the payload field 302 of the NDEF record 300, according to another embodiment. The payload field 302 of Figure 4 is used for communicating measurement data (e.g., physical health data) with a single touch. The payload field 302 encodes complete ISO/IEEE 11073-20601 personal health information, thereby enabling interoperability in exchanging ISO/IEEE 11073 personal health data. The payload field 302 helps reduce connection setup latency and communication overhead.

The payload field 302 includes a data proto-id field 402, a protocol version field 404, an encoding rules field 406, a nomenclature version field 408, a system identifier length field 410, a system identifier field 412, a config-report length field 414, a config-report data field 416, a measurement data length field 418, and a measurement data field 420.

The data proto-id field 402 indicates identifier of data exchange protocol. For example, the data proto-id field 402 includes a value '0', '20601' and '65535'. The value '20601' indicates IEEE 11073-20601 protocol is used. The protocol version field 404 is a one byte field indicating protocol identifier version used by the NFC agent 106. For example, the MSB 4 bits indicate a major release version and LSB 4 bits indicate a minor release version. The encoding rules field 406 indicates specific data APDU encoding rule(s) supported by the NFC agent 106. It is appreciated that, the NFC agent 106 and the NFC manager 102 supports Medical Device Encoding Rules (MDER) and negotiates on other encoding rule(s) except MDER. For example, the encoding rules field 406 includes a value '0' if MDER is supported, a value '1' if Extensible Markup Language Encoding Rules (XER) is supported, and a value '2' if Packed Encoding Rules (PER) is supported.

The nomenclature version field 408 indicates version of nomenclature used as defined in IEEE 11073-20601. For example, the MSB bit is set to a value '0' if the nomenclature version 1 is used. The system identifier length field 410 indicates length of the system identifier field 412. The system identifier field 412 includes a unique system identifier of the NFC agent 106. The EUI-64 format is used to identify the NFC agent 106. The system identifier field 412 enables the NFC manager 102 to determine identity of the NFC agent 106 and to implement a simple access restriction policy.

The config-report length field 414 indicates a length of the configuration report field 416. The conf-report data field 412 carries a configuration event report of the NFC agent 106 starting with object handling bytes. The format of the configuration-report data field 416 is similar to the configuration event report as specified in the ISO/IEEE 11073-20601 specification. The measurement data length 418 indicates length of the measurement data field 420. The measurement data field 420 carries measurement data report from the NFC agent 106 starting with object handle bytes. The format of the measurement data field 420 is similar to the measurement data report as specified in ISO/IEEE 11073-20601 specification.

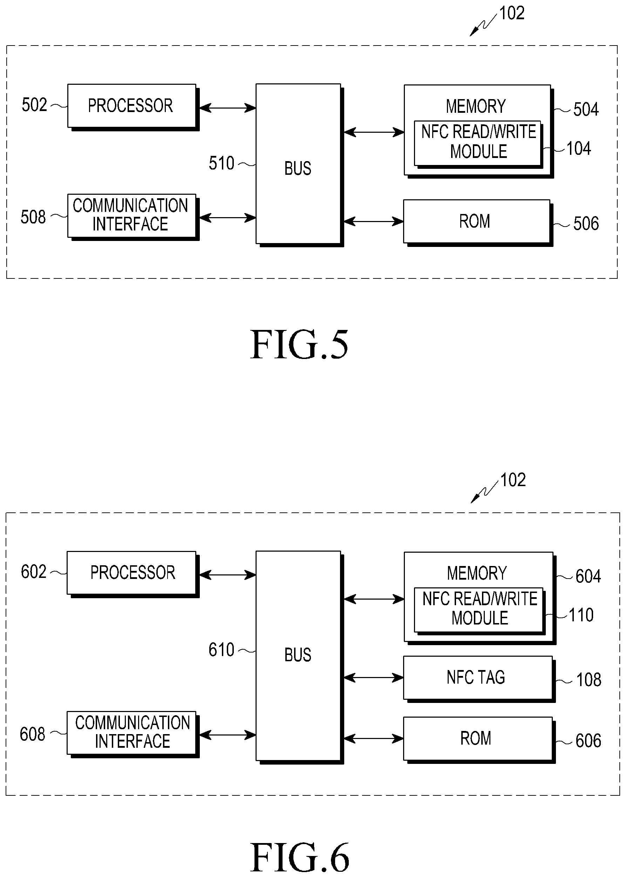

Figure 5 illustrates a block diagram of the NFC manager 102 showing various components for implementing embodiments of the present subject matter. In Figure 5, the NFC manager 102 includes a processor 502, memory 504, a read only memory (ROM) 506, a communication interface 508, and a bus 510.

The processor 502, as used herein, means any type of computational circuit, such as, but not limited to, a microprocessor, a microcontroller, a complex instruction set computing microprocessor, a reduced instruction set computing microprocessor, a very long instruction word microprocessor, an explicitly parallel instruction computing microprocessor, a graphics processor, a digital signal processor, or any other type of processing circuit. The processor 502 may also include embedded controllers, such as generic or programmable logic devices or arrays, application specific integrated circuits, single-chip computers, smart cards, and the like.

The memory 504 may be volatile memory and non-volatile memory. The memory 504 includes the NFC read/write module 104 for reading/writing NDEF records containing control information and other payload data from/to the NFC tag 108, according to the embodiments of the present invention. The communication interface may include 508 may be a radio frequency interface for enabling communication between the NFC Manager 102 and the NFC Agent 106 in a peer-to-peer mode, reader/writer mode, and a card emulation mode. The bus 510 enables communication between the various components of the NFC manager 102 illustrated therein.

Embodiments of the present subject matter may be implemented in conjunction with modules, including functions, procedures, data structures, and application programs, for performing tasks, or defining abstract data types or low-level hardware contexts. Machine-readable instructions stored on any of the above-mentioned storage media may be executable by the processor 502. For example, a computer program may include machine-readable instructions capable of reading/writing NDEF records containing control information and other payload data from/to the NFC tag 108, according to the teachings and herein described embodiments of the present subject matter. In one embodiment, the computer program may be included on a storage medium and loaded from the storage medium to a hard drive in the non-volatile memory.

Figure 6 illustrates a block diagram of the NFC agent 106 showing various components for implementing embodiments of the present subject matter. In Figure 6, the NFC agent 106 includes the NFC tag 108, a processor 602, memory 604, a read only memory (ROM) 606, a communication interface 608, and a bus 610.

The processor 602, as used herein, means any type of computational circuit, such as, but not limited to, a microprocessor, a microcontroller, a complex instruction set computing microprocessor, a reduced instruction set computing microprocessor, a very long instruction word microprocessor, an explicitly parallel instruction computing microprocessor, a graphics processor, a digital signal processor, or any other type of processing circuit. The processor 602 may also include embedded controllers, such as generic or programmable logic devices or arrays, application specific integrated circuits, single-chip computers, smart cards, and the like.

The memory 604 may be volatile memory and non-volatile memory. The memory 604 includes the NFC read/write module 104 for reading/writing NDEF records containing control information and other payload data from/to the NFC tag 108, according to the embodiments of the present invention. The communication interface may include 608 may be a radio frequency interface for enabling communication between the NFC manager102 and the NFC agent 106 in a peer-to-peer mode, reader/ writer mode, and a card emulation mode. The bus 610 enables communication between the various components of the NFC agent 106 illustrated therein.

Embodiments of the present subject matter may be implemented in conjunction with modules, including functions, procedures, data structures, and application programs, for performing tasks, or defining abstract data types or low-level hardware contexts. Machine-readable instructions stored on any of the above-mentioned storage media may be executable by the processor 602. For example, a computer program may include machine-readable instructions capable of reading/writing NDEF records containing control information and other payload data from/to the NFC tag 108 and communicating the NDEF records stored in the NFC tag into the NFC manger 102, according to the teachings and herein described embodiments of the present subject matter. In one embodiment, the computer program may be included on a storage medium and loaded from the storage medium to a hard drive in the non-volatile memory.

The various devices, modules, selectors, estimators, and the like described herein may be enabled and operated using hardware circuitry, for example, complementary metal oxide semiconductor based logic circuitry, firmware, software and/or any combination of hardware, firmware, and/or software embodied in a machine readable medium. For example, the various electrical structure and methods may be embodied using transistors, logic gates, and electrical circuits, such as application specific integrated circuit

The invention is specified in the appended claims.

## Claims

1. A method for transmitting and receiving data by a first near field communication, NFC, device (102) in NFC environment, the method comprising:
writing a first NFC data exchange format, NDEF, record including first control information into an NFC tag (108), wherein a value of a direction flag included in the first control information indicates that the first NDEF record in the NFC tag is sent from the first NFC device (102) to a second NFC device (106); and
reading (202) a second NDEF record including second control information from the NFC tag (108), wherein a value of a direction flag included in the second control information indicates that the second NDEF record in the NFC tag is sent from the second NFC device (106) to the first NFC device (102).

2. The method of claim 1, further comprising:
repeating the steps of writing and reading until a communication between the first NFC device (102) and the second NFC device (106) is completed.

3. The method of claim 2, wherein the second NDEF record includes a payload field carrying the second control information and the payload data.

4. The method of claim 3, wherein the payload data includes the personal health data.

5. The method of claim 1, wherein each of the first control information and the second control information further includes a request/response type flag indicating whether payload data is an ISO/IEEE 11073-20601 protocol request command, or an ISO/IEEE 11073-20601 protocol response command.

6. The method of claim 1, wherein each of the first control information and the second control information further includes a state flag indicating a state of a sender of a corresponding NDEF record.

7. The method of claim 6, wherein the state includes a disconnected state, a connected state, an unassociated state, an associating state, an associated state, an operating state, a configuring state, or a disassociating state.

8. A first near field communication, NFC, device (102) for transmitting and receiving data in NFC environment, the NFC device (102) comprising:
a processor (502); and
a memory (504) coupled to the processor (502), wherein the memory comprises a read/write module (104) configured to:
write a first NFC data exchange format, NDEF, record including the first control information into an NFC tag (108), wherein a value of a direction flag included in the first control information indicates that the first NDEF record in the NFC tag is sent from the first NFC device (102) to a second NFC device (106), and
read a second NDEF record including second control information from the NFC tag (108), wherein a value of a direction flag included in the second control information indicates that the second NDEF record in the NFC tag is sent from the second NFC device (106) to the first NFC device (102).

9. The first NFC device (102) of claim 8, wherein the second NDEF record further includes a payload field carrying the second control information and the payload data.

10. The first NFC device (102) of claim 9, wherein the payload data includes personal health data.

11. The first NFC device (102) of claim 8, wherein each of the first control information and the second control information further includes a request/response type flag indicating whether payload data is at least one of an ISO/IEEE 11073-20601 protocol request command or an ISO/IEEE 11073-20601 protocol response command.

12. The first NFC device (102) of claim 8, wherein each of the first control information and the second control information further includes a state flag indicating a state of a sender of a corresponding NDEF record.

13. The first NFC device (102) of claim 12, wherein the state includes a disconnected state, a connected state, an unassociated state, an associating state, an associated state, an operating state, a configuring state, or a disassociating state.

## Patentansprüche

1. Verfahren zum Übertragen und Empfangen von Daten durch eine erste Nahfeldkommunikations(NFC)-Vorrichtung (102) in einer NFC-Umgebung, wobei das Verfahren Folgendes umfasst:
Schreiben eines ersten NFC-Datenaustauschformat(NDEF)-Datensatzes einschließlich erster Kontrollinformationen in ein NFC-Tag (108), wobei ein Wert eines Richtungsflags, das in den ersten Kontrollinformationen enthalten ist, anzeigt, dass der erste NDEF-Datensatz in dem NFC-Tag von der ersten NFC-Vorrichtung (102) an eine zweite NFC-Vorrichtung (106) gesendet wird; und
Lesen (202) eines zweiten NDEF-Datensatzes einschließlich zweiter Kontrollinformationen von dem NFC-Tag (108), wobei ein Wert eines Richtungsflags, das in den zweiten Kontrollinformationen enthalten ist, anzeigt, dass der zweite NDEF-Datensatz in dem NFC-Tag von der zweiten NFC-Vorrichtung (106) an die erste NFC-Vorrichtung (102) gesendet wird.

2. Verfahren nach Anspruch 1, welches ferner Folgendes umfasst:
Wiederholen der Schritte des Schreibens und Lesens, bis eine Kommunikation zwischen der ersten NFC-Vorrichtung (102) und der zweiten NFC-Vorrichtung (106) abgeschlossen ist.

3. Verfahren nach Anspruch 2, wobei der zweite NDEF-Datensatz ein Nutzlastfeld enthält, das die zweiten Kontrollinformationen und die Nutzlastdaten trägt.

4. Verfahren nach Anspruch 3, wobei die Nutzlastdaten die persönlichen Gesundheitsdaten enthalten.

5. Verfahren nach Anspruch 1, wobei jede der ersten Kontrollinformationen und der zweiten Kontrollinformationen ferner ein Anforderungs-/Antworttyp-Flag enthält, das anzeigt, ob die Nutzlastdaten ein ISO/IEEE 11073-20601-Protokollanforderungsbefehl oder ein ISO/IEEE 11073-20601-Protokollantwortbefehl sind.

6. Verfahren nach Anspruch 1, wobei jede der ersten Kontrollinformationen und der zweiten Kontrollinformationen ferner ein Zustandsflag enthält, das einen Zustand eines Absenders eines entsprechenden NDEF-Datensatzes anzeigt.

7. Verfahren nach Anspruch 6, wobei der Zustand einen getrennten Zustand, einen verbundenen Zustand, einen nicht-assoziierten Zustand, einen assoziierenden Zustand, einen assoziierten Zustand, einen Betriebszustand, einen Konfigurationszustand oder einen dissoziierenden Zustand enthält.

8. Eine erste Nahfeldkommunikations(NFC)-Vorrichtung (102) zum Übertragen und Empfangen von Daten in einer NFC-Umgebung, wobei die NFC-Vorrichtung (102) Folgendes umfasst:
einen Prozessor (502); und
einen Speicher (504), der mit dem Prozessor (502) gekoppelt ist, wobei der Speicher ein Lese-/Schreibmodul (104) umfasst, das konfiguriert ist zum:
Schreiben eines ersten NFC-Datenaustauschformat(NDEF)-Datensatzes einschließlich der ersten Kontrollinformationen in ein NFC-Tag (108), wobei ein Wert eines Richtungsflags, das in den ersten Kontrollinformationen enthalten ist, anzeigt, dass der erste NDEF-Datensatz in dem NFC-Tag von der ersten NFC-Vorrichtung (102) an eine zweite NFC-Vorrichtung (106) gesendet wird, und
Lesen eines zweiten NDEF-Datensatzes einschließlich zweiter Kontrollinformationen von dem NFC-Tag (108), wobei ein Wert eines Richtungsflags, das in den zweiten Kontrollinformationen enthalten ist, anzeigt, dass der zweite NDEF-Datensatz in dem NFC-Tag von der zweiten NFC-Vorrichtung (106) an die erste NFC-Vorrichtung (102) gesendet wird.

9. Erste NFC-Vorrichtung (102) nach Anspruch 8, wobei der zweite NDEF-Datensatz ferner ein Nutzlastfeld enthält, das die zweiten Kontrollinformationen und die Nutzlastdaten trägt.

10. Erste NFC-Vorrichtung (102) nach Anspruch 9, wobei die Nutzlastdaten persönliche Gesundheitsdaten enthalten.

11. Erste NFC-Vorrichtung (102) nach Anspruch 8, wobei jede der ersten Kontrollinformationen und der zweiten Kontrollinformationen ferner ein Anforderungs-/Antworttyp-Flag enthält, das anzeigt, ob die Nutzlastdaten ein ISO/IEEE 11073-20601-Protokollsanforderungsbefehl und/oder ein ISO/IEEE 11073-20601-Protokollantwortbefehl sind.

12. Erste NFC-Vorrichtung (102) nach Anspruch 8, wobei jede der ersten Kontrollinformationen und der zweiten Kontrollinformationen ferner ein Zustandsflag enthält, das einen Zustand eines Absenders eines entsprechenden NDEF-Datensatzes anzeigt.

13. Erste NFC-Vorrichtung (102) nach Anspruch 12, wobei der Zustand einen getrennten Zustand, einen verbundenen Zustand, einen nicht-assoziierten Zustand, einen assoziierenden Zustand, einen assoziierten Zustand, einen Betriebszustand, einen Konfigurationszustand oder einen dissoziierenden Zustand enthält.

## Revendications

1. Procédé de transmission et de réception de données par un premier dispositif (102) de communication en champ proche, NFC, dans un environnement de NFC, le procédé comprenant :
écrire un premier dossier en format d'échange de données de NFC, NDEF, incluant des premières informations de commande dans une étiquette de NFC (108), où une valeur d'un indicateur de direction inclus dans les premières informations de commande indique que le premier dossier en NDEF dans l'étiquette de NFC est envoyé depuis le premier dispositif de NFC (102) à un deuxième dispositif de NFC (106) ; et
lire (202) un deuxième dossier en NDEF incluant des deuxièmes informations de commande en provenance de l'étiquette de NFC (108), où une valeur d'un indicateur de direction inclus dans les deuxièmes informations de commande indique que le deuxième dossier en NDEF dans l'étiquette de NFC est envoyé depuis le deuxième dispositif de NFC (106) au premier dispositif de NFC (102).

2. Procédé selon la revendication 1, comprenant en outre :
répéter les étapes d'écriture et de lecture jusqu'à ce qu'une communication entre le premier dispositif de NFC (102) et le deuxième dispositif de NFC (106) soit achevé.

3. Procédé selon la revendication 2, où le deuxième dossier en NDEF comprend un champ de charge utile portant les deuxièmes informations de commande et les données de charge utile.

4. Procédé selon la revendication 3, où les données de charge utile comprennent les données de santé personnelles.

5. Procédé selon la revendication 1, où chacune des premières informations de commande et des deuxièmes informations de commande comprend en outre un indicateur de type de demande/réponse indiquant si des données de charge utile constituent une commande de demande de protocole ISO/IEEE 11073-20601 ou une commande de réponse de protocole ISO/IEEE 11073-20601 .

6. Procédé selon la revendication 1, où chacune des premières informations de commande et des deuxièmes informations de commande comprend en outre un indicateur d'état indiquant un état d'un expéditeur d'un dossier en NDEF correspondant.

7. Procédé selon la revendication 6, où l'état comprend un état déconnecté, un état connecté, un état non associé, un état d'association, un état associé, un état de fonctionnement, un état de configuration ou un état de dissociation.

8. Premier dispositif de communication en champ proche, NFC, (102) permettant de transmettre et de recevoir des données dans un environnement de NFC, le dispositif de NFC (102) comprenant :
un processeur (502) ; et
une mémoire (504) couplée au processeur (502), où la mémoire comprend un module de lecture/écriture (104) configuré pour :
écrire un premier dossier en format d'échange de données de NFC, NDEF, incluant les premières informations de commande dans une étiquette de NFC (108), où une valeur d'un indicateur de direction inclus dans le premières informations de commande indique que le premier dossier en NDEF dans l'étiquette de NFC est envoyé depuis le premier dispositif de NFC (102) à un deuxième dispositif de NFC (106), et
lire un deuxième dossier en NDEF incluant des deuxièmes informations de commande en provenance de l'étiquette de NFC (108), où une valeur d'un indicateur de direction inclus dans les deuxièmes informations de commande indique que le deuxième dossier en NDEF dans l'étiquette de NFC est envoyé depuis le deuxième dispositif de NFC (106) au premier dispositif de NFC (102).

9. Premier dispositif de NFC (102) selon la revendication 8, où le deuxième dossier en NDEF comprend en outre un champ de charge utile portant les deuxièmes informations de commande et les données de charge utile.

10. Premier dispositif de NFC (102) selon la revendication 9, où les données de charge utile comprennent des données de santé personnelles.

11. Premier dispositif de NFC (102) selon la revendication 8, où chacune des premières informations de commande et des deuxièmes informations de commande comprend en outre un indicateur de type de demande/réponse indiquant si des données de charge utile constituent au moins l'une parmi une commande de demande de protocole ISO/IEEE 11073-20601 ou une commande de réponse de protocole ISO/IEEE 11073-20601.

12. Premier dispositif de NFC (102) selon la revendication 8, où chacune des premières informations de commande et des deuxièmes informations de commande comprend en outre un indicateur d'état indiquant un état d'un expéditeur d'un dossier en NDEF correspondant.

13. Premier dispositif de NFC (102) selon la revendication 12, où l'état comprend un état déconnecté, un état connecté, un état non associé, un état d'association, un état associé, un état de fonctionnement, un état de configuration ou un état de dissociation.
